# EUROPEAN PATENT SPECIFICATION

(11) **EP 0 422 215 B1**
(45) Date of publication and mention of the grant of the patent: **19.08.1998**
(21) Application number: 90908061.6
(22) Date of filing: 12.04.1990
(51) Int. Cl.: A61K 38/16, A61K 39/00, C07K 14/00

(54) **ANTIBACTERIAL AND ANTIMALARIAL PEPTIDES**
ANTIBAKTERIELLE- UND ANTIMALARIAPEPTIDE
PEPTIDES ANTIBACTERIENS ET ANTIPALUDEENS

(30) Priority: 12.04.1989 US 336777; 12.12.1989 US 449593
(43) Date of publication of application: 17.04.1991
(73) Proprietor: THE ROCKEFELLER UNIVERSITY, New York, NY 10021 (US)
(72) Inventor: BOMAN, Hans, G., S-11424 Stockholm (SE); MERRIFIELD, Robert, Bruce, Creskill, NJ 07626 (US)
(74) Representative: Weinhold, Peter, Dr.
(86) International application number: US9002082
(87) International publication number: WO9011771

(56) References cited:
- EP-A- 0 257 656
- US-A- 4 355 104
- CHEMICAL ABSTRACTS, Volume 109, Number 19, 11 February 1988, Columbus, Ohio, US; J.M. JAYNES: "Method for Introduction of Disease and Pest Resistance Into Plants and Novel Genes Incorporated Into Plants Which Code Therefor", see page 222, column 1, Abstract Number 165036y, PCT Int. App. WO 88 00, 976, 25 July 1986
- CHEMICAL ABSTRACTS, Volume 112, Number 7, 1990, Columbus, Ohio, US; J.M. JAYNES: "Plants Genetically Enhanced for Disease-Resistance or Enhanced Nutritional Quality", see page 455, column 2, Abstract Number 52535d, PCT Int. App. WO 88 04, 371, 18 May 1989
- CHEMICAL ABSTRACTS, Volume 111 Number 11, 1989, (Columbus, Ohio, USA) J. FINK, "The Chemical Synthesis of Creropin D and an Analog with Enhanced Antibacterial Activity", see page 794, column 1 and 2, Abstract Number 97703c, J. Biol. Chem., 1989, 264(11), 6260-7 (Eng).
- CHEMICAL ABSTRACTS, Volume 111, Number 25, 1989, Columbus, Ohio, US; R. W. GWADZ: "Effects of Magainins and Cecropins on the Sporogonic Development of Malaria Parasites in Mosquitoes", see page 17, column 2, Abstract Number 224848f, Infect. Immun., 1989, 57(a), 2628-33 (Eng)
- JOURNAL OF THE AMERICAN SOCIETY vol. 103, no. 3, 1981, GASTON, PA US pages 679 - 681; DEGRADO, W.F. ET AL.: 'Design, Synthesis and Charactarization of a Cytotoxic Peptide with Melittin-like Activity'
- FEBS LETTERS vol. 269, no. 1, 18 December 1989, AMSTERDAM NL pages 103 - 106; BOMAN, H.G. ET AL.: 'Antibacterial and Antimaterial Properties of Peptides that are Cecropin-Melittin Hybrids'

## Description

Several naturally occurring, antibiotically active peptides with useful therapeutic activity against pathogenic bacteria and other classes of microorganisms have recently been identified and isolated from insects, frogs and other animals. These include cecropins, attacins, magainins, sarcotoxin, sapecin, bactenecins, alamethicins, defensins and PGLa.

Other naturally occuring peptides from microorganisms, from insects and from higher animals are generally known as toxins that lyse red blood cells as well as other eukaryotic cells. These toxins include different hemolysins such as streptolysins, melittin, barbatolysin, paradaxins and delta hemolysin. It is known but it is not widely recognized that some toxins like melittin will also kill bacteria. Therefore, for purposes of this description they will be described as antibiotically active peptides.

The invention described and claimed herein is based on the unexpected discovery that novel antibiotic molecules can be constructed by joining together at least two sequences from different antibiotics. One advantage of such hybrid molecules is that they may be constructed to be shorter and therefore easier to synthesize than the natural peptides from which they are derived.

The hybrid peptides, in addition to their antibiotic activity, appear to have other features in common. For example they all contain about 20 to 40 amino acids and often they are more effective if their C-terminals are amidated or blocked in other ways. They are therefore potential candidates for commercial preparation by solid phase synthesis. Additionally, they all appear to contain certain sequences of amino acids which impart specific secondary characteristics to portions of the molecule. Often the N-terminus is hydrophilic and basic, and the C-terminus is hydrophobic. Some portions of the molecule have a tendency towards helicity, others do not. Some molecules contain relatively long sequences which are flexible, thus forming hinge regions in the molecule. Often the helical portions are amphipathic, i.e., they are characterized by a hydrophilic and a hydrophobic surface.

The antibiotics appear to function by rupturing the cell membrane of the bacteria or other organism. Binding of the peptides to the membrane permits entry of ions into the cellular fluid thus increasing the osmotic pressure and causing more fluid to enter the cell which increases the internal pressure and force the cell to burst. The differing secondary characteristics of the different portions of the antibiotic peptides appear to be associated with their mode of action in piercing the cell membrane.

A very important current medical problem is to find antibiotics with enhanced potency against human pathogens especially those for which no suitable antibiotic is now available or to which resistant organisms have emerged by mutation of the original pathogenic organisms. One response to the emergence of organisms resistant to antibiotics has been to prepare synthetic derivatives of these compounds, but this approach has been limited by the availability of functional groups on the parent molecule that can be utilized as foci for preparing derivatives.

It would be useful if a pool of antibiotics of comparatively simple structure were available which could be synthesized with relative ease and which at the same time would be susceptible to a variety of readily accomplished structural variations to produce synthetic antibiotically active peptides useful against specific organisms for which no non-toxic antibiotic is presently available, or of improved activity against other organisms for which the presently available antibiotics are toxic to the host. Such compounds should also have sufficient in vivo stability to resist degradation by mammalian enzymes.

It has been discovered that naturally occurring peptides such as those mentioned above, and others like them constitute such a pool.

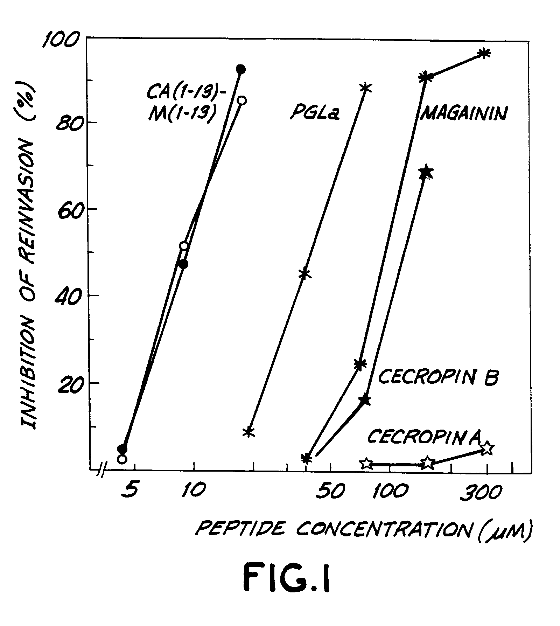

Fig. 1 shows peptide concentration (µM) as a function of inhibition of reinvasion for assays tested for activity against Plasmodium falciparum.

The present invention provides antibiotically and/or antimalarially active, non-toxic synthetic hybrid peptides containing from 20 to 40 amino acid residues and comprising at least one region containing from 5 to 20 amino acid residues in a sequence corresponding to a sequence on a naturally occuring antibiotically and/or antimalarially active peptide selected from cecropin (C), cecropin A (CA), cecropin B (CB), cecropin D (CD), melittin (M), magainin (Mag), or attacin, said region being combined with one or more other regions of the same or another naturally occuring antibiotically and/or antimalarially active peptide.

The present invention also provides pharmaceutical compositions containing said peptides.

The peptides of the invention are of improved pharmaceutical activity for any of a number of reasons. Some have improved activity against known pathogens. Some, unlike their naturally occurring counterparts are non-toxic and do not cause lysis of red blood cells. Still others are active against pathogens for which no completely satisfactory treatment is presently available.

Preferred therapeutically useful peptides of this invention are characterized as hybrids containing at least one amino acid region containing from about 10 to about 15 amino acid residues in a sequence which is substantially similar to a corresponding segment on a natural antibiotic peptide, although variations in the length and sequence of the amino acid residues are possible.

This invention will be better understood by consideration of the application of the invention to cecropins and melittin.

The cecropins are a family of basic antibacterial peptides produced by the humoral immune response of certain insects as described in US-A-4,355,104. Cecropins, together with attacins and lysozyme, are induced in the hemolymph of the pupae of the giant silk moth Hylophora cecropia following injection of live bacteria. There are three major cecropins, A, B and D. There is a high degree of homology between them, and all are of about the same size (cecropin A: 37 residues, cecropin B: 35 residues, cecropin D: 36 residues). They each contain a hydrophilic amino terminal chain and a hydrophobic amidated carboxyl terminus.

The amino acid sequences of cecropin A, B and D are shown in Table 1 which also includes the sequence of melittin. For convenience and ease of analysis the cecropin molecules have been divided into three sections: residues 1-11, 12-24 and 25 to the end. Those skilled in the art will recognize the high degree of homology and that cecropins A and B will be quite similar in their secondary structures. Both would be expected to have a strong potential in a polar environment to form an N-terminal amphipathic α-helix. The C-terminus will also have a tendency towards α-helix formation. In the central segment 12-24, there is some tendency for β-turns, for instance at residues 12-15, 15-18, 21-24. The N-terminus of cecropin D is less basic than either A or B. However, the central region of cecropin D has a higher potential for an α-helix than the A and B forms and also a strong preference for a helix in the C-terminal region.

In summary, the cecropins have a strongly hydrophilic amphipathic α-helix at the N-terminus, a more hydrophobic α-helix at the C-terminus, and a rather flexible, structurally less defined central region with some potential for β-turns.

The structure of melittin, an antibacterial peptide isolated from bee venom is shown in Table 1. It is a cationic amphipathic peptide in which residues 1-20 are predominantly hydrophobic and residues 21 to 26 are hydrophilic and basic. It will be noted that the major regions are arranged opposite from the cecropins. In melittin, they are hydrophobic/hydrophilic, and in the cecropins they are hydrophilic/hydrophobic. In the middle of the molecule there is a Gly-Leu-Pro region which may act as a hinge. Melittin has antibiotic activity, but is not useful for mammals because it is lytic for leukocytes, red blood cells and a wide variety of other cells.

It has now been discovered that it is possible to improve the pharmaceutical utility of naturally occurring, low molecular weight, antibiotically active peptides by rearranging selected regions or sequences of the peptides or, in some instances, adding a wholly new region to an intact region of a naturally occurring peptide. Thus a peptide formed by uniting the first 13 amino acid residues of cecropin A as the amino terminus with the first 13 amino acid residues of melittin, CA(1-13) M(1-13), has a lower lethal concentration and is more active against Staphylococcus aureus or Bacillus subtilis than cecropin A. Additionally, the novel 26 residue peptide is not lytic to sheep red blood cells even at concentrations greater than 200 µM. In contrast, melittin is lytic at 4-6 µM.

The term "improving the pharmaceutical activity" as used herein means that the novel peptide is less toxic to mammalian cells and/or more active against a spectrum of pathogens or against a specific pathogen than a naturally occurring peptide from which it is derived. A peptide of the invention is said to be "derived" from a naturally occurring peptide if it contains at least one region which is identical or substantially homologous to a region on a naturally occurring peptide. Thus CA(1-13) M(1-13) can be considered as derived from both cecropin A and melittin. Other peptides within the scope of the invention may contain, for example, sequences from a magainin and an attacin, rearranged regions of one antibiotic, for example melittin, or one region from a cecropin and another wholly unnatural region designed to be more or less hydrophobic, hydrophilic, or helical than the "naturally occurring" sequence.

It will be apparent to those skilled in the art that the selected region of the "naturally occurring" peptide in the final novel products of the invention does not need to be identical with the region in the natural peptide. One or more of the amino acid residues of the natural peptide may be replaced with another selected amino acid to increase the basicity, to interrupt the helicity or for any other useful reason. The sequence of amino acid residues in the novel product will, however, be substantially similar to the natural sequence.

The peptides of the invention will normally contain from about 20 to about 40 amino acid residues. One reason is that antibiotically active low molecular weight peptides usually contain a minimum of about 20 amino acids. Another, is that peptides with more than about 40 amino acids are relatively difficult to synthesize in pure form by chemical synthesis, and may be best prepared by fermentation or recombinant DNA procedures. A particular advantage of the useful peptides of this invention is that they are readily synthesized by solid phase methods and a variety of combinations are possible to achieve specifically required results. An advantage of the use of solid phase techniques is that the product can be directly synthesized with the C-terminus amidated or otherwise blocked.

The term "region" as used herein is similar to "segment" or "fragment". It refers to amino acid sequences containing at least 5 and normally from about 5 to about 20 amino acids. A "region" is usually selected or constructed to be flexible, basic, hydrophobic, hydrophilic, amphipathic or helical, and that will be a characteristic of the region. A molecule may be constructed to have at least two regions and may or may not contain a hinge region. The region does not need to be derived from a naturally occurring antibiotically active peptide containing 20 to 40 amino acid residues. It may be derived from a peptide containing less than 20 or more than 40 such residues.

The invention, then, comprises antibiotically and/or antimalarially active peptides of improved antibiotic and/or antimalarial activity containing from about 20 to about 40 amino acid residues including at least one region with from 5 to 20 amino acid residues which is substantially similar to a corresponding sequence on a naturally occurring antibiotically and/or antimalarially active peptide from which the novel peptide is derived, and may be combined with one or more other regions of the same or another natural antibiotic and/or antimalarial peptide to form a hybrid molecule.

In another aspect, the invention is a method for improving the pharmaceutical activity of antibiotically and/or antimalarially active peptides which comprises synthesizing novel peptides containing from about 20 to about 40 amino acid residues containing at least one amino acid sequence or region containing from about 5 to 20 amino acid residues which is substantially identical to a corresponding sequence on a naturally occurring antibiotically and/or antimalarially active peptide.

Typical compounds within the scope of this invention may be represented by the following list wherein C represents cecropin, CA, CB and CD represent the A, B and D forms of cecropin, M represents melittin and Mag represents magainin. The numbers represent the sequence of amino acid residues in the corresponding region of the natural peptide. The notations define the characteristics of the region
- CA(1-13)Mag(13-23) -: hydrophilic/hydrophobic
- M(15-26)Mag(13-23) -: hydrophilic/hydrophobic
- Mag(13-23)CA(1-13) -: hydrophobic/hydrophilic
- Mag(13-23)M(15-26) -: hydrophobic/hydrophilic
- M(1-13)CB(1-13) -: hydrophobic/hydrophilic
- M(1-12)ProCA(1-13) -: hydrophobic-Prohydrophilic
- M(1-15)C(1-11) -: hydrophobic/hydrophilic
- M(16-26)CA(14-37) -: hydrophilic/hydrophobic
- CA(25-36)ProCA(1-13) -: hydrophobic-Prohydrophilic
- CA(25-37)CA(1-13) -: hydrophobic/hydrophilic
- CA(1-24)M(1-13) -: hydrophilic/hydrophobic
- CA(1-13)M(1-13) -: hydrophobic/hydrophilic
- M(16-26)M(1-13) -: hydrophilic/hydrophobic
- M(16-26)CA(23-37) -: hydrophilic/hydrophobic
- CA(1-24)M(16-26) -: hydrophilic/hydrophilic
- CB(25-35)M(14-26) -: hydrophobic/hydrophilic
- CA(1-11)CD(12-37) -: hydrophilic/hydrophobic
- CA(1-8)M(1-18) -: hydrophilic/hydrophobic
- CA(1-9)M(1-17) -: hydrophilic/hydrophobic
- CB(1-13)M(1-13) -: hydrophilic/hydrophobic
- CA(1-17)M(1-9) -: hydrophilic/hydrophobic
- CA(1-18)M(1-8) -: hydrophilic/hydrophobic
- M(1-13)CA(1-22) -: hydrophobic/hydrophilic
- M(1-13)CA(1-13) -: hydrophobic/hydrophilic

Most of the above products, in addition to being hydrophobic/hydrophilic or vice-versa will also have regions of helicity or amphipathicity. Proline (Pro) is often employed to interrupt a helix although other amino acids may be similarly employed. The above peptides can also be constructed to include a flexible or hinge region.

The compounds of this invention are synthesized by standard solid phase procedures using the protection, deprotection and cleavage techniques and reagents appropriate to each specific amino acid or peptide. A combination of manual and automated (e.g., Applied Biosystem 430A) solid phase techniques can be used to synthesize the novel peptides of this invention. For background on solid pahse techniques, reference is made to Andreu, D., Merrifield, R. B., Steiner, H. and Boman, H.G., (1983) Proc. Natl. Acad. Sci USA 80, 6475-6479; Andreu, D., Merrifield, R.B., Steiner, H. and Boman, H.G., (1985) Biochemistry 24, 1683-1688; Fink, J., Boman, A., Boman, H.G., and Merrifield, R.B., (June 1989) Int. J. Peptide Protein Res. 33, 412-421; Fink, J., Merrifield, R.B., Boman, A. and Boman, H.G., (1989) J. Biol. Chem. 264, 6260-6267.

The in vivo stability of the compounds of the invention can be improved by adding a D-amino acid to the N- and C-termini.

Since the products of the invention are amphoteric they may be utilized as free bases, as acid addition salts or as metal salts. The salts must, of course, be pharmaceutically acceptable, and these will include metal salts, particularly alkali and alkaline earth metal salts, suitably potassium or sodium salts. A wide variety of pharmaceutically acceptable acid addition salts are available. These include those prepared from both organic and inorganic acids, preferably mineral acids. Typical acids which may be mentioned by way of example include citric, succinic, lactic, hydrochloric and hydrobromic acids. Such products are readily prepared by procedures well known to those skilled in the art.

A further aspect of the present invention provides pharmaceutical compositions which comprise one or more compounds of the invention and a pharmaceutically acceptable carrier. The compositions may be made up of any pharmaceutical form appropriate for the desired route of administration. Examples of such compositions include solid compositions for oral administration such as tablets, capsules, pills, powders and granules. Liquid compositions for oral administration such as solutions, suspensions, syrups or elixirs and preparations for parenteral administration such as sterile solutions, suspensions or emulsions. The compositions may also be manufactured in the form of sterile solid compositions which can be dissolved in sterile water, physiological saline or some other sterile injectable medium immediately before use. Topical compositions, typically in the form of emulsions, suspensions, creams, lotions or foams which may contain emolients, suspending agents, chelating agents, stiffening agents, buffering agents, and other components conventionally used with topical compositions containing antibiotics may also be provided.

In all such compositions the antibiotic and/or antimalarial will normally be the principal physiologically active ingredient.

Optimal dosages and regimens for a given mammalian host can be readily ascertained by those skilled in the art. It will, of course, be appreciated that the actual dose used will vary according to the particular composition formulated, the particular compound used, the mode of application and the particular site, host and disease being treated. Many factors that modify the action of the drug will be taken into account including age, weight, sex, diet, time of administration, route of administration, rate of excretion, condition of the patient, drug combinations, reaction sensitivities and severity of the disease.

The following non-limiting examples are given by way of illustration only and are not to be considered limitations of this invention, many apparent variations of which are possible without departing from the spirit or scope thereof.

### EXAMPLES

### EXAMPLES 1-6 AND COMPARISON WITH CA(1-37), CD(2-37), AND M(1-26)

Novel peptides of this invention and melittin were synthesized by a combination of automated (Applied Biosystem 430A) solid phase techniques (see articles regarding synthesis, cited supra). In particular, melittin and the novel peptides CA(25-37)CA(1-13), M(1-13)CA(1-13), CA(1-11)CD(12-37), CA(1-24)M(1-13), CA(1-13)M(1-13), and M(16-26)M(1-13) were prepared by the following standard double coupling protocol, based on 2.5 g of starting resin (0.21 mmol/g): (1) CH₂Cl₂, 50 mL, 4 x 1 min; (2) 50% TFA/CH₂Cl₂, 50 mL, 2 x 1 min; (3) 50% TFA/CH₂Cl₂, 50 mL, 1 x 20 min; (4) CH₂Cl₂, 50 mL, 6 x 1 min; (5) 5% DIEA/CH₂Cl₂, 50 mL, 2 x 2 min; (6) CH₂Cl₂, 50 mL, 6 x 1 min; (7) protected amino acid, 4 eq in 20 mL of CH₂Cl₂, add to reaction vessel, rinse with 4 mL of CH₂Cl₂, and shake at room temperature for 5 min; 4 eq of DCC in 3 mL of CH₂Cl₂, add to reaction vessel, rinse with 2 mL of CH₂Cl₂ and shake for 100 min at room temperature; (8) CH₂Cl₂, 50 mL, 4 x 1 min; (9) 5% DIEA/CH₂Cl₂, 50 mL, 1 x 2 min; (10) CH₂Cl₂, 50 mL, 4 x 1 min; (11) DMF, 50 mL, 2 x 2 min; (12) protected amino acid, 8 eq in 3 mL of CH₂Cl₂, 0°C, add DCC, 4 eq in 1 mL of CH₂Cl₂, 0°C, rinse with 1 mL of CH₂Cl₂, 0°C, after 10 min. at 0°C, filter, add 25 mL of DMF, 0°C, add to reaction vessel, rinse with 5 mL of DMF, 0°C, shake for 1 h at room temperature; (13) DMF, 50 mL, 2 x 2 min; (14) CH₂Cl₂, 50 mL, 4 x 1 min; (15) 5% DIEA/CH₂Cl₂, 50 mL, 1 x 2 min; (16) CH₂Cl₂, 50 mL, 4 x 1 min; (17) 3- to 5-mg sample for ninhydrin analysis. This protocol is repeated for subsequent amino acids to complete the assembly of the desired peptide.

The fully protected peptide on the resin is then treated with TFA to remove the N-terminal Boc group and dried. Cleavage of the peptide from the resin support is accomplished by the low/high HF method, Tam et al (1983) J. Am. Chem. Soc. 105 6442-6455. Low HF is accomplished with 5 mL of HF/dimethyl sulfide/p-cresol/p-thiocresol (25:65:7.5:2.5), at 0°C for 2 hr. High HF is accomplished with 10 mL of HF/p-cresol/p-thiocresol (95:3.75:1.25), at 0°C for 1 hr. After evaporation of HF, the product is first washed with anhydrous ether to remove the scavengers, and then dissolved in 10% HOAc in water. The crude material is obtained by lyophilization.

The peptides of the invention are then partially purified by gel filtration on a Sephadex G-25 column in 1 M HOAc to remove low molecular weight impurities. After lyophilization the products are normally obtained in a yield of about 80%, based on the first amino acid. Reverse phase, low pressure, preparative liquid chromatography on a Vydac C18 column (218TPB10) and elution with a linear gradient of 25-65% acetonitrile, in certain instances, followed the Sephadex gel filtration.

The products of the invention were tested for activity against a variety of test organisms selected to represent a number of different types of pathogens some of them known to be particularly virulent by the inhibition zone assay of Hoffmann et al. (1981) Insect Biochem. 11 537-548. Thin agar or agarose plates (8.5 cm diameter) were prepared with 6 ml of rich medium containing 100 µg/ml of streptomycin and 2 x 10⁵ viable cells of a test organism resistant to streptomycin. Wells of 35mm diameter were punched in the plates and 3 µl of serially diluted samples were placed in the wells. The diameters of the zones of inhibition around the wells were measured after overnight incubation at 30° or 37°C. For each peptide the squares of the zone diameters were plotted against the logarithm of the concentration, and from the slopes and intercepts the lethal concentrations were calculated as described by Hultmark (1983) EMBO J. 2, 571-76 or Hultmark et al. (1982) Eur. J. Biochem. 127 207-217. The results of the assays with some of the products of the invention are shown in Table 2.

The Table 2 also shows the concentrations at which the compounds will lyse sheep red blood cells. These values were obtained by either the lysis assay normally used for melittin or by an adaptation of the antibacterial inhibition zone assay of the above-cited Hultmark et al. articles to plates with sheep red cells (SRC). With respect to the latter, sterile agarose plates contained 6 ml of medium with 1% agarose, 0.9% NaCl and 10% SRC suspended in Alsevers solution. A dilution series of peptide was applied in 3mm wells each loaded with 3 µl of the respective samples. The plates were incubated at 30°C for 24 h, the zones were recorded and LC values calculated as described in the Hultmark et al. articles. Clear zones were recorded after a few hours of incubation and the use of such data gave an LC value of the same order as readings after 24 h. This plate assay is faster and more convenient than the lysis assay normally used for melittin.

It was observed during testing that basic peptides often bind to agar plates, but not so much to agarose. Therefore those lethal concentrations determined on agar may, in fact, be substantially higher than the actual lethal concentrations.

It will be seen that all of the hybrid peptides are less toxic than melittin as measured by ability to lyse sheep red blood cells. It will be seen also that against specific organisms most of them are more active than at least one of the naturally occurring peptides from which it is derived. CA(1-11)CD(12-37) is more active than either cecropin A or cecropin D against the organisms tested. The most profound increases in antibiotic activity were with CA(1-13)M(1-13) and CA(1-24)M(1-13) against Staphylococcus aureus and Bacillus subtilis where increases of 40 to 200 fold over cecropin A were observed. The potency of these two hybrid peptides against a yeast Saccharomyces cerevisiae was also greater than cecropin A. Furthermore, these two compounds were not toxic to sheep red blood cells even at 100 µM and higher.

One interesting embodiment of this invention is the hybrid M(16-26)M(1-13) in which the two principal regions of melittin are reversed. This product did not lyse red cells even at 240 µM, whereas the natural melittin molecule caused lysis at 4-6 µM, a greater than 40-60 fold improvement.

### EXAMPLES 7-8 and COMPARISON WITH Mag, CB, CA and PGLa

Hybrid peptides CA(1-13)M(1-13) and CA(1-8)M(1-18) were prepared as described above (see Examples 1-6) and were assayed for activity against the blood stream form of Plasmodium falciparum. For comparison the test included cecropins A and B and the frog skin peptides magainin 2 and PGLa.

The blood stream forms of the malaria parasite Plasmodium falciparum, primarily late trophozoites and early schizonts, were assayed by recording the inhibition of the reinvasion of human red cells by the method of Wahlin, B., et al, (1984) Proc. Natl. Acad. Sci. USA 81, 7912-16. Quadruplicate microcultures of strain F32 (Tanzania) were incubated for 20 hrs at 37°C in a complete tissue culture medium, with or without different concentrations of the peptides to be assayed. After acridine orange staining, the percentage of newly infected red cells was scored in a fluorescence microscope. For each culture 40,000 red cells were analysed for parasitic infection. Duplicate assays were run for CA(1-13)M(1-13).

Figure 1 shows the results for CA(1-13)M(1-13), CA, CB, Mag and PGLa. From Figure 1, CA has only negligible activity; CB is about as potent as Mag; hybrid CA(1-13)M(1-13) is of an order more potent than CB; and, PGLa has intermediate activity. Duplicate assays were run for CA(1-13)M(1-13). CA(1- 8)M(1-18) is even more potent than CA(1-13)M(1-13). CA(1-8)M(1-18) is in fact about four times more potent than CA(1-13)M(1-13) : CA(1-8)M(1-18), at a concentration of approximately 2.2 µM yields a 50% inhibition of reinvasion. Only a trace of hemolytic activity was observed with the hybrids of the present invention.

### EXAMPLES 9-15 AND COMPARISON WITH CA, CB AND M

Novel peptides CA(1-8)M(1-18), CA(1-9)M(1-17), CB(1-13)M(1-13), CA(1-17)M(1-9), CA(1-18)M(1-8), M(1-13)CA(1-22) and M(1-13)CA(1-13) were prepared as described above (see Examples 1-6). These novel peptides and CA, CB, and M were tested for activity against a variety of organisms selected to represent a number of different types of pathogens, some of them known to be particularly virulent, by the inhibition zone assay of Hoffmann et al. (1981), Insect Biochem. 11 537-48. As in Examples 1-6, thin agarose plates were prepared with the respective organisms. The diameters of the zones of inhibition were measured after overnight incubation at 30°C. The lethal concentrations were determined as described above (see Examples 1-6; Hultmark et al. articles cited thereat). The results of the assays of these novel peptides are shown in Table 3.

As seen from Table 3, the hybrid peptides are less toxic than melittin; and, are more active than at least one of the naturally occurring peptides from which it is derived.

**TABLE 1**

| Cercropin A: |
|---|
| H-Lys-Trp-Lys-Leu-Phe-Lys-Lys-Ile-Glu-Lys-Val-Gly-Gln-Asn-Ile-Arg-Asp-Gly-Ile-Ile-Lys-Ala-Gly-Pro-Ala-Val-Ala-Val-Val-Gly-Gln-Ala-Thr-Gln-Ile-Ala-Lys-NH₂ |

| Cecropin B: |
|---|
| H-Lys-Trp-Lys-Val-Phe-Lys-Lys-Ile-Glu-Lys-Met-Gly-Arg-Asn-Ile-Arg-Asn-Gly-Ile-Val-Lys-Ala-Gly-Pro-Ala-Ile-Ala-Val-Leu-Gly-Glu-Ala-Lys-Ala-Leu-NH₂ |

| Cecropin D: |
|---|
| H-Trp-Asn-Pro-Phe-Lys-Glu-Leu-Glu-Lys-Val-Gly-Gln-Arg-Val-Arg-Asp-Ala-Val-Ile-Ser-Ala-Gly-Pro-Ala-Val-Ala-Thr-Val-Ala-Gln-Ala-Thr-Ala-Leu-Ala-Lys-NH₂ |

| Melittin: |
|---|
| H-Gly-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln(NH₂) |

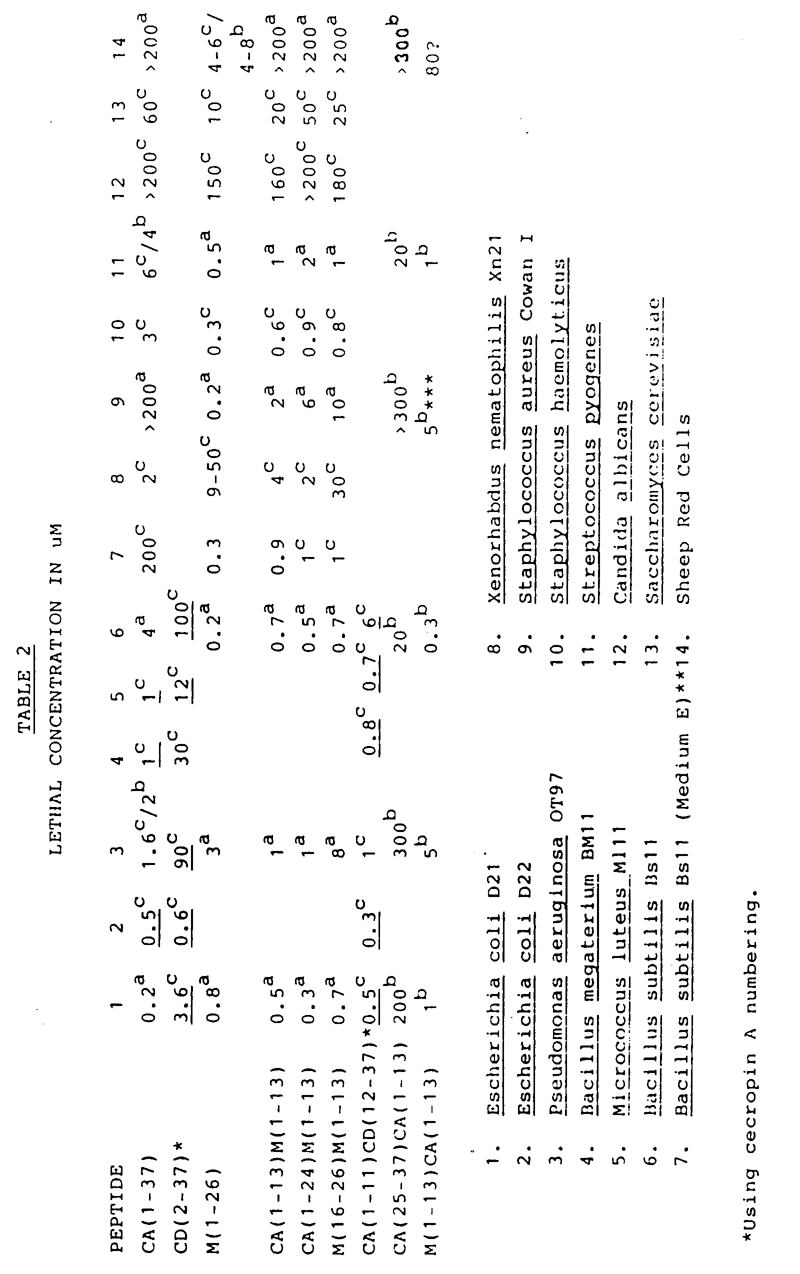

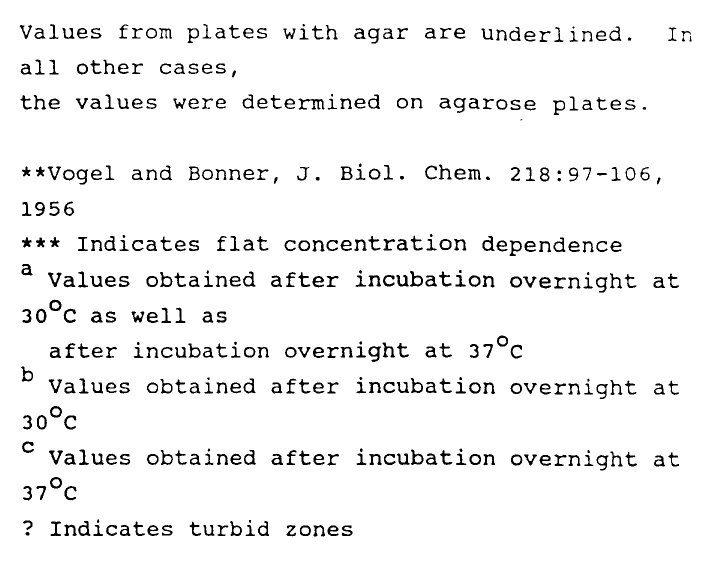

**TABLE 3**

| LETHAL CONCENTRATION IN µM | | | | | | | | |
|---|---|---|---|---|---|---|---|---|
| Compound | Size (aa) | D21 | OT97 | Bsll | Bsll + ME | Staph | Strep | SRC |
| CA(1-37) | 37 | 0.2 | 2 | 4 | 200 | >200 | 4 | >200 |
| CB(1-35) | 35 | 0.3 | 1 | nd | nd | >200 | 12 | nd |
| M(1-26) | 26 | 0.8 | 3 | 0.2 | 0.3 | 0.2 | 0.5 | 4-8 |
| CA(1-8)M(1-18) | 26 | 0.3 | 0.7 | 0.4 | 0.5 | 1 | 2 | >600 |
| CA(1-9)M(1-17) | 26 | 0.3 | 1 | 0.7 | 0.9 | 6 | 0.3 | >600 |
| CB(1-13)M(1-13) | 26 | 0.3 | 1 | 0.4 | 0.4 | 4 | 1 | >400 |
| CA(1-17)M(1-9) | 26 | 0.5 | 5 | 0.5 | 1 | 4 | 0.2 | >600 |
| CA(1-18)M(1-8) | 26 | 1 | 15 | 4 | 20 | >600 | 3 | >600 |
| M(1-13)CA(1-22) | 35 | 0.7 | nd | nd | 0.2 | 5* | 1 | 40? |
| M(1-13)CA(1-13) | 26 | 1 | 5 | 0.3 | 0.1 | 5* | 1 | 80? |
| D21 = E. coli OT97 = P. aeruginosa Bsll = B. subtilis Bsll + ME = B. subtilis, plates prepared with medium E Staph = S. aureus Cowan I Strep = S. pyogenes SRC = Sheep Red Cells nd = not determined | | | | | | | | |

| | | | | | | | | |
|---|---|---|---|---|---|---|---|---|
| ? indicates turbid zone (melittin gives clear zone) | | | | | | | | |
| * indicates flat concentration dependence | | | | | | | | |

## Claims

1. Antibiotically and/or antimalarially active, synthetic hybrid peptides containing from 20 to 40 amino acid residues and comprising at least one region containing from 5 to 20 amino acid residues in a sequence corresponding to a sequence on a naturally occuring antibiotically and/or antimalarially active peptide selected from cecropin (C), cecropin A (CA), cecropin B (CB), cecropin D (CD), melittin (M), magainin (Mag), or attacin, said region being combined with one or more other regions of the same or another naturally occuring antibiotically and/or antimalarially active peptide.

2. A peptide of claim 1 containing two regions each containing from 10 to 15 amino acid residues in a sequence corresponding to a sequence on a naturally occurring antibiotically and/or antimalarially active peptide selected from cecropin (C), cecropin A (CA), cecropin B (CB), cecropin D (CD), melittin (M), magainin (Mag), or attacin.

3. A peptide of claim 2 wherein both of the regions are from the same naturally occurring antibiotically and/or antimalarially active peptide.

4. A peptide of claim 1 comprising CA(1-13)M(1-13), CA(1-24)M(1-13), CA(1-11)CD(12-37) or M(16-26)M(1-13).

5. A peptide of claim 1 selected from the group consisting of CA(1-13)Mag(13-23), M(15-26)Mag(13-23), Mag(13-23)CA(1-13), Mag(13-23)M(15-26), M(1-13)CB(1-13), M(1-12)ProCA(1-13), M(1-15)C(1-11), CA(25-36)ProCA(1-13), CA(25-37)CA(1-13), CA(1-13)M(1-13), M(16-26)M(1-13), M(16-26)CA(23-37), CB(25-35)M(14-26), CB(1-13)M(1-13), M(1-13)CA(1-13), M(16-26)CA(14-37), CA(1-24)M(1-13), CA(1-24)M(16-26), CA(1-11)CD(12-37), CA(1-8)M(1-18), CA(1-9)M(1-17), CA(1-17)M(1-9), CA(1-18)M(1-8), and M(1-13)CA(1-22).

6. A pharmaceutical composition containing a pharmaceutically acceptable carrier together with one or more antibiotically and/or antimalarially active, synthetic hybrid peptides containing from 20 to 40 amino acid residues and comprising at least one region containing from 5 to 20 amino acid residues in a sequence corresponding to a sequence on a naturally occurring antibiotically and/or antimalarially active peptide selected from cecropin (C), cecropin A (CA), cecropin B (CB), cecropin D (CD), melittin (M), magainin (Mag), or attacin, said region being combined with one or more other regions of the same or another naturally occuring antibiotically and/or antimalarially active peptide.

7. A composition of claim 6 wherein the peptide is one which contains two regions each containing from 10 to 15 amino acid residues in a sequence corresponding to a sequence on a naturally occurring antibiotically and/or antimalarially active peptide selected from cecropin (C), cecropin A (CA), cecropin B (CB), cecropin D (CD), melittin (M), magainin (Mag), or attacin.

8. A composition of claim 7, wherein the peptide is one in which both of the regions are from the same naturally occurring antibiotically and/or antimalarially active peptide.

9. A composition of claim 6 wherein the peptide is selected from one or more of CA(1-24)M(1-13), CA(1-11)CD(12-37), M(16-26)M(1-13), M(16-26)CA(14-37), CA(1-24)M(1-13), CA(1-24)M(16-26), CA(1-11)CD(12-37), CA(1-8)M(1-18), CA(1-9)M(1-17), CA(1-17)M(1-9), CA(1-18)M(1-8), M(1-13)CA(1-22), CA(1-13)Mag(13-23), M(15-26)Mag(13-23), Mag(13-23)CA(1-13), Mag(13-23)M(15-26), M(1-13)CB(1-13), M(1-12)ProCA(1-13), M(1-15)C(1-11), CA(25-36)ProCA(1-13), CA(25-37)CA(1-13), CA(1-13)M(1-13), M(16-26)M(1-13), M(16-26)CA(23-37), CB(25-35)M(14-26), CB(1-13)M(1-13), and M(1-13)CA(1-13).

10. A process for preparing the peptides according to any of claims 1-5, wherein the peptides are synthesized by solid phase technique.

11. Use of the peptides of any of claims 1-5 for preparing a pharmaceutical composition having antibiotic and/or antimalarial activity.

## Patentansprüche

1. Antibiotisch und/oder gegen Malaria wirkende, synthetische Hybridpeptide, die von 20 bis 40 Aminosäurereste enthalten und mindestens einen Bereich aufweisen, welcher von 5 bis 20 Aminosäurereste in einer Sequenz enthält, die einer Sequenz auf einem natürlich vorkommenden, antibiotisch und/oder gegen Malaria wirkenden Peptid entspricht, das aus Cecropin (C), Cecropin A (CA), Cecropin B (CB), Cecropin D (CD), Melittin (M), Magainin (Mag) oder Attacin ausgewählt ist, wobei dieser Bereich mit einem oder mehr Bereichen des gleichen oder eines weiteren natürlich vorkommenden, antibiotisch und/oder gegen Malaria wirkenden Peptids kombiniert ist.

2. Peptid nach Anspruch 1, welches zwei Bereiche enthält, die jeweils von 10 bis 15 Aminosäurereste in einer Sequenz enthalten, welche einer Sequenz auf einem natürlich vorkommenden, antibiotisch und/oder gegen Malaria wirkenden Peptid entspricht, das aus Cecropin (C), Cecropin A (CA), Cecropin B (CB), Cecropin D (CD), Melittin (M), Magainin (Mag) oder Attacin ausgewählt ist.

3. Peptid nach Anspruch 2, bei dem beide der Bereiche von dem gleichen natürlich vorkommenden, antibiotisch und/oder gegen Malaria wirkenden Peptid sind.

4. Peptid nach Anspruch 1, welches CA(1-13)M(1-13), CA(1-24)M(1-13), CA(1-11)CD(12-37) oder M(16-26)M(1-13) aufweist.

5. Peptid nach Anspruch 1, ausgewählt aus der Gruppe bestehend aus CA(1-13)Mag(13-23), M(15-26)Mag(13-23), Mag(13-23)CA(1-13), Mag(13-23)M(15-26), M(1-13)CB(1-13), M(1-12)ProCA(1-13), M(1-15)C(1-11), CA(25-36)ProCA(1-13), CA(25-37)CA(1-13), CA(1-13)M(1-13), M(16-26)M(1-13), M(16-26)CA(23-37), CB(25-35)M(14-26), CB(1-13)M(1-13), M(1-13)CA(1-13), M(16-26)CA(14-37), CA(1-24)M(1-13), CA(1-24)M(16-26), CA(1-11)CD(12-37), CA(1-8)M(1-18), CA(1-9)M(1-17), CA(1-17)M(1-9), CA(1-18)M(1-8) und M(1-13)CA(1-22).

6. Pharmazeutische Zusammensetzung, welche einen pharmazeutisch akzeptablen Träger zusammen mit einem oder mehr antibiotisch und/oder gegen Malaria wirkenden, synthetischen Hybridpeptiden enthält, welche von 20 bis 40 Aminosäurereste enthalten und mindestens einen Bereich aufweisen, der von 5 bis 20 Aminosäurereste in einer Sequenz enthält, welche einer Sequenz auf einem natürlich vorkommenden, antibiotisch und/oder gegen Malaria wirkenden Peptid entspricht, das aus Cecropin (C), Cecropin A (CA), Cecropin B (CB), Cecropin D (CD), Melittin (M), Magainin (Mag) oder Attacin ausgewählt ist, wobei dieser Bereich mit einem oder mehr weiteren Bereichen des gleichen oder eines weiteren natürlich vorkommenden, antibiotisch und/oder gegen Malaria wirkenden Peptids kombiniert ist.

7. Zusammensetzung nach Anspruch 6, bei der das Peptid ein solches ist, das zwei Bereiche enthält, die jeweils von 10 bis 15 Aminosäurereste in einer Sequenz enthalten, die einer Sequenz auf einem natürlich vorkommenden, antibiotisch und/oder gegen Malaria wirkenden Peptid entspricht, das aus Cecropin (C), Cecropin A (CA), Cecropin B (CB), Cecropin D (CD), Melittin (M), Magainin (Mag) oder Attacin ausgewählt ist.

8. Zusammensetzung nach Anspruch 7, bei der das Peptid ein solches ist, bei dem beide der Bereiche von dem gleichen natürlich vorkommenden, antibiotisch und/oder gegen Malaria wirkenden Peptid sind.

9. Zusammensetzung nach Anspruch 6, bei der das Peptid aus einem oder mehr von CA(1-24)M(1-13), CA(1-11)CD(12-37), M(16-26)M(1-13), M(16-26)CA(14-37), CA(1-24)M(1-13), CA(1-24)M(16-26), CA(1-11)CD(12-37), CA(1-8)M(1-18), CA(1-9)M(1-17), CA(1-17)M(1-9), CA(1-18)M(1-8), M(1-13)CA(1-22), CA(1-13)Mag(13-23), M(15-26)Mag(13-23), Mag(13-23)CA(1-13), Mag(13-23)M(15-26), M(1-13)CB(1-13), M(1-12)ProCA(1-13), M(1-15)C(1-11), CA(25-36)ProCA(1-13), CA(25-37)CA(1-13), CA(1-13)M(1-13), M(16-26)M(1-13), M(16-26)CA(23-37), CB(25-35)M(14-26), CB(1-13)M(1-13) und M(1-13)CA(1-13) ausgewählt ist.

10. Verfahren zur Bereitung der Peptide nach einem der Ansprüche 1-5, bei dem die Peptide mittels der Festphasentechnik synthetisiert werden.

11. Verwendung der Peptide nach einem der Ansprüche 1-5 zur Bereitung einer pharmazeutischen Zusammensetzung mit antibiotischer Wirkung und/oder Wirkung gegen Malaria.

## Revendications

1. Peptides hybrides synthétiques, actifs en tant qu'antibiotiques et/ou antipaludéens, contenant de 20 à 40 résidus d'acides aminés et comprenant au moins une région contenant de 5 à 20 résidus d'acides aminés dans une séquence qui correspond à une séquence sur un peptide naturel, actif en tant qu'antibiotique et/ou antipaludéen, choisi parmi la cécropine (C), la cécropine A (CA), la cécropine B (CB), la cécropine D (CD), la mélittine (M), la magaïnine (Mag), ou l'attacine, ladite région étant combinée avec une ou plusieurs autres régions du même peptide ou d'un autre peptide naturel actif en tant qu'antibiotique et/ou antipaludéen.

2. Peptide selon la revendication 1, contenant deux régions contenant chacune de 10 à 15 résidus d'acides aminés dans une séquence qui correspond à une séquence sur un peptide naturel, actif en tant qu'antibiotique et/ou antipaludéen, choisi parmi la cécropine (C), la cécropine A (CA), la cécropine B (CB), la cécropine D (CD), la mélittine (M), la magaïnine (Mag), ou l'attacine.

3. Peptide selon la revendication 2, dans lequel les deux régions sont issues du même peptide naturel actif en tant qu'antibiotique et/ou antipaludéen.

4. Peptide selon la revendication 1, comprenant CA(1-13)M(1-13), CA(1-24)M(1-13), CA(1-11)CD(12-37) ou M(16-26)M(1-13).

5. Peptide selon la revendication 1, choisi dans le groupe constitué par
CA(1-13)Mag(13-23), M(15-26)Mag(13-23), Mag(13-23)CA(1-13), Mag(13-23)M(15-26), M(1-13)CB(1-13), M(1-12)ProCA(1-13), M(1-15)C(1-11), CA(25-36)ProCA(1-13), CA(25-37)CA(1-13), CA(1-13)M(1-13), M(16-26)M(1-13), M(16-26)CA(23-37), CB(25-35)M(14-26), CB(1-13)M(1-13), M(1-13)CA(1-13), M(16-26)CA(14-37), CA(1-24)M(1-13), CA(1-24)M(16-26), CA(1-11)CD(12-37), CA(1-8)M(1-18), CA(1-9)M(1-17), CA(1-17)M(1-9), CA(1-18)M(1-8), et M(1-13)CA(1-22).

6. Composition pharmaceutique contenant un véhicule pharmaceutiquement acceptable ainsi qu'un ou plusieurs peptides hybrides synthétiques actifs en tant qu'antibiotiques et/ou antipaludéens, contenant de 20 à 40 résidus d'acides aminés et comprenant au moins une région contenant de 5 à 20 résidus d'acides aminés dans une séquence qui correspond à une séquence sur un peptide naturel, actif en tant qu'antibiotique et/ou antipaludéen, choisi parmi la cécropine (C), la cécropine A (CA), la cécropine B (CB), la cécropine D (CD), la mélittine (M), la magaïnine (Mag), ou l'attacine, ladite région étant combinée avec une ou plusieurs autres régions du même peptide ou d'un autre peptide naturel actif en tant qu'antibiotique et/ou antipaludéen.

7. Composition selon la revendication 6, dans laquelle le peptide est un peptide qui contient deux régions contenant chacune de 10 à 15 résidus d'acides aminés dans une séquence qui correspond à une séquence sur un peptide naturel, actif en tant qu'antibiotique et/ou antipaludéen, choisi parmi la cécropine (C), la cécropine A (CA), la cécropine B (CB), la cécropine D (CD), la mélittine (M), la magaïnine (Mag), ou l'attacine.

8. Composition selon la revendication 7, dans laquelle le peptide est un peptide dans lequel les deux régions sont issues du même peptide naturel actif en tant qu'antibiotique et/ou antipaludéen.

9. Composition selon la revendication 6, dans laquelle le peptide est choisi parmi un ou plusieurs des CA(1-24)M(1-13), CA(1-11)CD(12-37), M(16-26)M(1-13), M(16-26)CA(14-37), CA(1-24)M(1-13), CA(1-24)M(16-26), CA(1-11)CD(12-37), CA(1-8)M(1-18), CA(1-9)M(1-17), CA(1-17)M(1-9), CA(1-18)M(1-8), M(1-13)CA(1-22), CA(1-13)Mag(13-23), M(15-26)Mag(13-23), Mag(13-23)CA(1-13), Mag(13-23)M(15-26), M(1-13)CB(1-13), M(1-12)ProCA(1-13), M(1-15)C(1-11), CA(25-36)ProCA(1-13), CA(25-37)CA(1-13), CA(1-13)M(1-13), M(16-26)M(1-13), M(16-26)CA(23-37), CB(25-35)M(14-26), CB(1-13)M(1-13), et M(1-13)CA(1-13).

10. Procédé de préparation des peptides selon l'une quelconque des revendications 1-5, dans lequel les peptides sont synthétisés par une technique en phase solide.

11. Utilisation des peptides selon l'une quelconque des revendications 1-5, pour préparer une composition pharmaceutique ayant une activité antibiotique et/ou antipaludéenne.
